(19) Europäisches Patentamt
European Patent Office
Office européen des brevets

(11) Veröffentlichungsnummer: **0 173 832**
**B1**

(12) **EUROPÄISCHE PATENTSCHRIFT**

(45) Veröffentlichungstag der Patentschrift: **25.01.89**

(51) Int. Cl.[4]: **C 07 C 141/08**, C 07 C 143/12, C 11 D 1/16, C 25 D 3/22

(21) Anmeldenummer: **85108799.9**

(22) Anmeldetag: **13.07.85**

(54) **Sulfate und Sulfonderivate der Beta - Naphtholpolyglykoläther und saure Zinkbäder enthaltend diese Verbindungen.**

(30) Priorität: **06.09.84 DE 3432956**

(43) Veröffentlichungstag der Anmeldung: **12.03.86 Patentblatt 86/11**

(45) Bekanntmachung des Hinweises auf die Patenterteilung: **25.01.89 Patentblatt 89/4**

(84) Benannte Vertragsstaaten: **BE CH DE FR GB IT LI LU NL SE**

(56) Entgegenhaltungen: **GB-A-2 054 554**
**US-A-4 075 066**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Voss, Günter, Am Rosenanger 30, D-1000 Berlin 28 (DE)**
Erfinder: **Seidenspinner, Hubert- Matthias, Dr., Attendorner Weg 15, D-1000 Berlin 27 (DE)**

**EP 0 173 832 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue Sulfate und Sulfoderivate der β-Naphtholpolyglykoläther gemäß Oberbegriff des Anspruchs 1 und saure Zinkbäder enthaltend diese Verbindungen gemäß Oberbegriff des Anspruchs 7.

Die Verwendung von sulfonierten und/oder sulfatierten Alkylphenolethoxylaten in sauren galvanischen Zinkbädern ist bereits bekannt (DE-OS-32 458 503). Derartige Bäder zeigen den Nachteil, beim Einblasen von Luft eine störende Schaumschicht zu entwickeln, wodurch eine einwandfreie Metallabscheidung verhindert wird.

Es ist weiterhin bekannt, polyoxyalkylierte Naphthole neben anionischen Sulfonsäurekondensaten und Sulfonsäuren sowie nichtionogenen Sulfonsäurekondensaten in sauren Zinkbädern zu verwenden (US-PS-4 075 066). Ein Nachteil dieser Bäder ist es, daß im Laufe der Durcharbeit der Trübungspunkt sinkt und durch Überdosierung bestimmter Netzmittel angehoben werden muß.

Aus der GB-PS-2 054 554 sind schließlich Ammoniumsalze von Sulfaten der Beta-Naphtholpolyglykoläther als Additive für galvanische Zinnbäder bekannt geworden. Eine Lehre zur Verwendung dieser Verbindungen in sauren galvanischen Zinkbädern ist dieser Druckschrift nicht zu entnehmen.

Aufgabe der vorliegenden Erfindung ist die Schaffung neuer Tenside und diese Produkte enthaltende saure Zinkbader mit hohem Trübungspunkt, welche eine einwandfreie Metallabscheidung ohne Schaumentwicklung ermöglichen.

Diese Aufgabe wird erfindungsgemäß durch die Bereitstellung von Sulfaten und Sulfoderivaten der β-Naphtholpolyglykoläther und eines sauren Zinkbades, das mindestens eine solche Verbindung enthalt, gelöst.

Diese Sulfate und Sulfoderivate der 82-Naphtholpolyglykoläther besitzen die allgemeine Formel

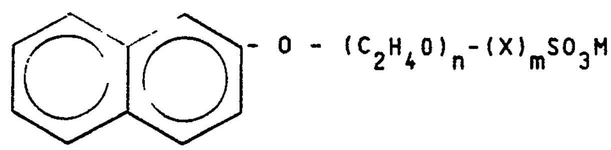 I

in der

X eine gegebenenfalls substituierte divalente $C_1$-$C_6$-Alkylengruppe oder eine gegebenenfalls substituierte divalente Phenylengruppe,

M Wasserstoff, ein Alkalimetallatom oder ein entsprechendes Äquivalent eines zweiwertigen Metalls,

n eine ganze Zahl von 6 bis 60 und

m 0 oder 1 bedeuten.

Diese Verbindungen zeichnen sich überraschenderweise durch eine außerordentlich geringe Schaumentwicklung bei gleichzeitig hervorragender grenzflachenartiger Wirkung aus, worin sie bekannten Tensiden gleicher Wirkungsrichtung überlegen sind.

Die erfindungsgemäßen Verbindungen eignen sich insbesondere als Zusätze zu sauren Zinkbädern, die außer den üblichen Bestandteilen hochwirksame Glanzbildner enthalten.

Als solche Glanzbildner seien insbesondere aromatische Ketone und aromatische Aldehyde, wie zum Beispiel Benzalaceton und o-Chlorbenzaldehyd, genannt, die aufgrund ihrer geringen Löslichkeit in derartigen Bädern in der Regel in Gegenwart von gewissen oberflächenaktiven Stoffen eingesetzt werden.

Bekannte Stoffe dieser Art neigen jedoch zur Schaumbildung oder zeigen den Nachteil, in sauren Zinkbädern unbefriedigend zu wirken, was den Trübungspunkt derartiger Bäder stark herabsetzt.

Die erfindungsgemäßen Verbindungen überwinden die genannten Nachteile des Standes der Technik. Saure Zinkbäder, welche die erfindungsgemäßen Verbindungen enthalten, entwickeln auch beim Einblasen von Luft keine störende Schaumschicht, was sie für den Einsatz in modernen Hochleistungselektrolyten besonders geeignet macht. Als weitere Vorteile sind herauszustellen der stabile und hohe Trübungspunkt der erfindungsgemäßen Bäder sowie die Qualität der hieraus abgeschiedenen Metallüberzüge, die sich durch schleierfreien Glanz auszeichnen.

Als Verbindungen mit herausragender Wirkung seien insbesondere β-Naphtholpolyglykoläthersulfosuccinat mit 15 Äthylenoxydgruppen und β-Naphtholpolyglykoläthersulfosuccinat mit 24 Äthylenoxydgruppen genannt.

Die erfindungsgemäßen Verbindungen werden in Mengen von etwa 0,5 bis 20 g/Liter, vorzugsweise 4 bis 10 g/Liter, verwendet.

Als Badlösung wird im allgemeinen eine wässrige Lösung von Zinkchlorid und Kaliumchlorid folgender Konzentration benutzt:

| | |
|---|---|
| Zinkchlorid ($ZnCl_2$) | 30 - 120 g/Liter |
| Kaliumchlorid (KCl) | 60 - 200 g/Liter |

Statt Zinkchlorid können zumindest teilweise auch andere Zinksalze benutzt werden, wie zum Beispiel Zinksulfat, Zinkacetat, Zinkfluoroborat und/oder Zinksulfamat. Kaliumchlorid kann teilweise oder ganz durch Ammoniumchlorid, Ammoniumsulfat oder andere Leitsalze, die Natriumchlorid, Natriumsulfat oder ähnliche ersetzt werden. Schließlich kann das Bad aliphatische und/oder aromatische Carbonsäuren beziehungsweise

deren Salze, wie zum Beispiel Benzoesäure, Salicylsäure oder Naphthalsäure, enthalten.

Als weitere Zusatzstaffe können Borsäure oder andere Puffergemische sowie nichtschäumende Netzmittel enthalten sein.

Die Arbeitsbedingungen sind wie folgt:

| | |
|---|---|
| pH-Wert | : 3,0 - 6,0 vorzugsweise 4,5 - 5,5 |
| Temperatur | : 15 - 40° C vorzugsweise 20 - 30° C |
| Stromdichte (kathodisch) | : 0,1 - 10,0 A/$dm^2$ |

Elektrolytbewegung vorzugsweise durch Lufteinblasen und/oder Kathodenstangenbewegung.

Die Herstellung der erfindungsgemäßen Verbindungen erfolgt in an sich bekannter Weise

a) durch Sulfatierung der entsprechenden β-Naphtholpolyglykoläther oder

b) durch Veresterung der entsprechenden β-Naphtholpolyglykoläther, zum Beispiel mit Maleinsäure oder Maleinsäureanhydrid und nachfolgende Umsetzung mit Bisulfit beziehungsweise Metabisulfit.

Die folgenden Beispiele dienen zur Erläuterung der Anwendungsmöglichkeiten der erfindungsgemäßen Verbindungen.

Die Ausführung der Beispiele erfolgte in einer Hullzelle unter den angegebenen Bedingungen.

Verwendet wurde die jeweils aufgeführte Elektrolytlösung, der die in den Beispielen angeführten Zusätze zugelöst waren.

**Beispiel 1**

| | |
|---|---|
| Zinkchlorid | 72,0 g/Liter |
| Kaliumchlorid | 155,0 g/Liter |
| Borsäure | 23,0 g/Liter |
| Benzoesäure | 2,0 g/Liter |
| Benzalaceton | 0,12 g/Liter |

<u>a) mit erfindungsgemäßem Zusatz</u>

| | |
|---|---|
| β-Naphtholpolyglykoläther-sulfosuccinat mit 15 Äthylenoxydgruppen | 8,0 g/Liter |

b) mit bekanntem Zusatz (gemäß <u>US-PS-4 075 066)</u>

| | |
|---|---|
| β-Naphtholpolyglykoläther mit 15 Äthylenoxydgruppen | 8,0 g/Liter |

c) mit bekanntem Zusatz (gemäß <u>DE-OS-3 248 503)</u>

| | |
|---|---|
| Sulfatierter Nonylphenolpolyglykoläter- | 8,0 g/Liter |

<u>Durchführung</u>

| | |
|---|---|
| Zellstrom | 2 A |
| Temperatur | 22° C |
| Lufteinblasen Dauer : | 10 Minuten |

Die verzinkten Eisenbleche wurden anschließend zur Hälfte blauchromatiert.

<u>Ergebnisse</u>

a) Badlösung : schäumt nicht
Trübungspunkt : > 100° C
Hochglänzender, duktiler Zinkniederschlag

b) Badlösung : schäumt nicht
Trübungspunkt : 26,5° C
Hochglänzender, duktiler Zinkniederschlag mit Anbrennungen bei hoher Stromdichte

c) Badlösung : schäumt
Trübungspunkt : ca. 80° C
Hochglänzender verschleierter, duktiler Zinkniederschlag mit Anbrennungen bei hoher Stromdichte

**Beispiel 2**

| | |
|---|---|
| Zinkchlorid | 72,0 g/Liter |
| Kaliumchlorid | 155,0 g/Liter |
| Borsäure | 23,0 g/Liter |
| Benzoesäure | 2,0 g/Liter |
| Benzalaceton | 0,1 g/Liter |
| Acetophenon | 0,02 g/Liter |

<u>a) mit erfindungsgemäßem Zusatz</u>

| | |
|---|---|
| β - Naphthopolyglykoläthersulfat mit 24 Äthylenoxydgruppen | 8,0 g/Liter |

b) mit bekanntem Zusatz (gemäß <u>DE-OS-3 248 503)</u>

| | |
|---|---|
| Nonylphenolpolyglykoläther mit 14 Äthylenoxydgruppen | 4,0 g/Liter |
| Sulfatierter Nonylphenolpolyglykoläther | 4,0 g/Liter |

<u>Durchführung</u>

| | |
|---|---|
| Zellstrom | 2 A |
| Temperatur | 22° C |
| Lufteinblasen Dauer: | 10 minuten |

Die verzinkten Eisenbleche wurden anschließend zur Hälfte blauchromatiert.

<u>Ergebnisse</u>

a) Badlösung schäumt nicht
Trübungspunkt : 75 g
Hochglänzender, duktiler Zinkniederschlag

b) Badlösung schäumt stark
Trübungspunkt : ca. 71° C
Hochglänzender, verschleierter, duktiler Zinkniederschlag mit Dendritenbildung im hohen Stromdichtebereich

**Beispiel 3**

| | |
|---|---|
| Zinkchlorid | 72,0 g/Liter |
| Kaliumchlorid | 155,0 g/Liter |
| Borsäure | 23,0 g/Liter |
| Benzoesäure | 0,08 g/Liter |
| o-Chlorbenzaldehyd | 0,04 g/Liter |
| Äthylhexanolpolyglykoläther | 2,0 g/Liter |
| mit 15 ÄthylenoxydgruppenCumolsulfonat-Natrium | 2,0 g/Liter |

<u>a) mit erfindungsgemäßem Zusatz</u>

| | |
|---|---|
| β-Naphtholpolyglykoläthersulfosuccinat mit 24 Äthylenoxdgruppen | 4,0 g/Liter |

b) mit bekanntem Zusatz (gemäß <u>US-PS-3 248 503)</u>

| | |
|---|---|
| β-Naphtholpolyglykoläther mit 15 Äthylenoxydgruppen | 4,0 g/Liter |

<u>Durchführung</u>

| | |
|---|---|
| Zellstrom | 2 A |
| Temperatur | 22° C |
| Lufteinblasen Dauer: | 10 Minuten |

Die verzinkten Eisenbleche wurden anschließend zur Hälfte blauchromatiert.

Ergebnisse

a) Badlösung schäumt nicht
Trübungspunkt : 88° C
Hochglänzender, duktiler Zinkniederschlag

b) Badlösung schäumt nicht
Trübungspunkt : 44° C (absinkend)
Hochglänzender verschleierter, duktiler Zinkniederschlag

**Beispiel 4**

| | |
|---|---|
| Zinkchlorid | 72,0 g/Liter |
| Kaliumchlorid | 155,0 g/Liter |
| Borsäure | 23,0 g/Liter |
| Benzoesäure | 2,0 g/Liter |
| Benzalaceton | 0,03 g/Liter |
| o-Chlorbenzaldehyd | 0,05 g/Liter |

a) mit erfindunsgemäßem Zusatz

| | |
|---|---|
| β-Naphtholpolyglykoläthersulfosuccinat mit 15 Äthylenoxydgruppen | 8,0 g/Liter |

Durchführung

| | |
|---|---|
| Zellstrom | 2 A |
| Temperatur | 22° C |
| Lufteinblasen Dauer : | 10 Minuten |

Die verzinkten Eisenbleche wurden anschließend zur Hälfte blauchromatiert.

Ergebnisse

a) Badlösung schäumt nicht
Trübungspunkt > 100° C
Hochglänzender, duktiler Zinkniederschlag

**Beispeil 4**

| | |
|---|---|
| Zinkchlorid | 72,0 g/Liter |
| Kaliumchlorid | 155,0 g/Liter |
| Borsäure | 23,0 g/Liter |
| Benzoesäure | 2,0 g/Liter |
| Benzalaceton | 0,03 g/Liter |
| o-Chlorbenzaldehyd | 0,05 g/Liter |

a) mit erfindungsgemäßem Zusatz

| | |
|---|---|
| β-Naphtholyglykoläthersulfat mit 12 Äthylenoxydgruppen | 8,0 g/Liter |

Durchführung

| | |
|---|---|
| Zellstrom | 2 A |
| Temperatur | 22° C |
| Lufteinblasen Dauer : | 10 Minuten |

Die verzinkten Eisenbleche wurden anschließend zur Hälfte blauchromatiert

Ergebnisse

Badlösung schmäumt nicht
Trübungspunkt > 100° C
Hochglänzender, duktiler Zinkniederschlag mit geringer Anbrennzone.

**Patentansprüche**

1. Sulfate und sulfoderivate der allgemeinen Formel

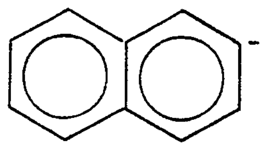 $- O - (C_2H_4O)_n-(X)_mSO_3M$ I

in der
X eine gegebenenfalls substituierte divalente $C_1$-$C_6$-Alkylengruppe oder eine gegebenenfalls substituierte divalente Phenylengruppe,
M Wasserstoff, ein Alkalimetallatom oder ein entsprechendes Äquivalent eines zweiwertigen Metalls,
n eine ganze Zahl von 6 bis 60 und
m 0 oder 1 bedeuten.

2. β-Naphtholpolyglykoläthersulfat mit 12 Äthylenoxydgruppen.
3. β-Naphtholpolyglykoläthersulfat mit 24 Äthylenoxydgruppen.
4. β-Naphtholpolyglykoläthersulfat mit 15 Äthylenoxydgruppen.
5. β-Naphtholpolyglykoläthersulfat mit 24 Äthylenoxydgruppen.
6. Verwendung der Verbindungen gemäß Ansprüchen 1 bis 5 als Tenside.
7. Saure Zinkbäder, gekennzeichnet durch einen Gehalt an mindestens einer Verbindung gemäß Ansprüchen 1 bis 5.
8. Saure Zinkbäder gemäß Anspruch 7 enthaltend Zinksalze, Leitsalze sowie übliche Glanzbildner.
9. Saure Zinkbäder gemäß Anspruch 8 enthaltend als Glanzbildner aromatische Ketone und/oder aromatische Aldehyde.

**Claims**

1. Sulfates and sulfo-derivatives of general formula

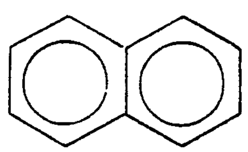 $- O - (C_2H_4O)_n-(X)_mSO_3M$ I

where
X is a possibly substituted divalent $C_1$-$C_6$ alkylene group or a possibly substituted divalent phenylene group,
M is hydrogen, an alkali metal atom or a corresponding equivalent of a divalent metal,
n is an integer number between 6 and 60, and
m is 0 or 1.

2. β-Naphtholpolyglycol-ether sulfate with 12 ethylene oxide groups.
3. β-Naphtholpolyglycol-ether sulfate with 24 ethylene oxide groups.
4. β-Naphtholpolyglycol-ether sulfosuccinate with 15 ethylene oxide groups.
5. β-Naphtholpolyglycol-ether sulfosuccinate with 24 ethylene oxide groups.
6. Utilization of the compounds in accordance with Claims 1 to 5 as surfactants.
7. Acid zinc baths, characterized in that they contain at least one of the compounds in accordance with Claims 1 to 5
8. Acid zinc baths in accordance with Claim 7, characterized in that they contain zinc salts, conductive salts as well as customary brighteners.
9. Acid zinc baths in accordance with Claim 8, characterized in that they contain aromatic ketones and/or aromatic aldehydes as brighteners.

## Revendications

1. Sulfates et dérivés sulfonés de la formule générale

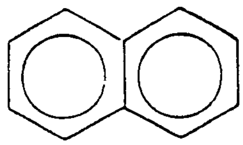 $-O-(C_2H_4O)_n-(X)_mSO_3M$ I

où
X signifie un groupe alkylène $C_1$-$C_6$ bivalent, substitué le cas échéant, ou un groupe phénylique bivalent, substitué le cas échéant,
M signifie hydrogène, un atome d'un métal alcalin ou un équivalent correspondant d'un métal bivalent,
n est un nombre entier entre 6 et 60, et
m est 0 ou 1.

2. Sulfate de l'éther β-naphtholpolyglycolique à 12 groupes d'oxyde d'éthylène.

3. Sulfate de l'éther β-naphtholpolyglycolique à 24 groupes d'oxyde d'éthylène.

4. Sulfosuccinate de l'éther β-naphthopolyglycolique à 15 groupes d'oxyde d'éthylène.

5. Sulfosuccinate de l'éther β-naphthopolyglycolique à 24 groupes d'oxyde d'éthylène.

6. Emploie des composés selon les revendications 1 à 5 en tant que surfactants.

7. Bains de zinc acides caractérisés en ce qu'ils contiennent au moins un des composés selon les revendications 1 à 5.

8. Bains de zinc acides selon revendication 7, caractérisés en ce qu'ils contiennent des sels zinciques, des sels conducteurs ainsi que des lustrants communs.

9. Bains de zinc acides selon revendication 8, caractérisés en ce qu'ils contiennent des cétones aromatiques et/ou des aldéhydes aromatiques en tant que lustrants.